# EUROPÄISCHE PATENTSCHRIFT

(11) **EP 2 838 414 B1**
(45) Veröffentlichungstag und Bekanntmachung des Hinweises auf die Patenterteilung: **02.12.2015**
(21) Anmeldenummer: 13732082.6
(22) Anmeldetag: 18.04.2013
(51) Int. Cl.: A61B 1/04

(54) **ROTATIONSVORRICHTUNG ZUM ROTIEREN EINES ENDOSKOPS**
ROTATIONAL DEVICE FOR ROTATING AN ENDOSCOPE
DISPOSITIF DE ROTATION D'UN ENDOSCOPE

(30) Priorität: 18.04.2012 DE 102012206412
(43) Veröffentlichungstag der Anmeldung: 25.02.2015
(73) Patentinhaber: Karl Storz GmbH & Co. KG, 78532 Tuttlingen (DE)
(72) Erfinder: OGINSKI, Stefan, 36041 Fulda (DE); BRÜGGEMANN, Daniel, 59556 Lippstadt (DE); DREYER GENANNT DAWEKE, Robert, 14109 Berlin (DE); RAMSCH, Christian, 13509 Berlin (DE)
(74) Vertreter: Wimmer, Stephan
(86) Internationale Anmeldenummer: PCT/DE2013/200010
(87) Internationale Veröffentlichungsnummer: WO 2013/156024

(56) Entgegenhaltungen:
- US-A1- 2010 185 212
- US-A1- 2011 288 374
- US-A1- 2012 065 470

## Beschreibung

Die Erfindung betrifft eine Rotationsvorrichtung zum Rotieren eines Endoskops.

Eine derartige Rotätionsvorrichtung umfasst eine rotierbare Baugruppe und eine feststehende Baugruppe sowie eine Antriebseinrichtung. An der rotierbaren Baugruppe ist eine distale Kopplungseinrichtung zum Halten eines Endoskops angeordnet. Die Antriebseinrichtung ist ausgebildet, die distale Kopplungseinrichtung in eine Drehbewegung zu versetzen, um ein mit der distalen Kopplungseinrichtung verbundenes Endoskop zu rotieren.

Eine Rotationsvorrichtung der hier beschriebenen Art kann insbesondere zum Rotieren eines medizinischen Endoskops verwendet werden, wie es insbesondere in mikroinvasiven Operationen an menschlichen oder tierischen Patienten eingesetzt wird. Ein solches Endoskop weist einen Schaft auf, der einem Operationsort zugeführt wird und unter anderem zum Übertragen von Licht von dem Operationsort hin zu einer Kameravorrichtung dient. Mittels der Kameravorrichtung wird das Licht erfasst und in ein analoges oder digitales Signal gewandelt, das zu einer Anzeigevorrichtung übertragen wird, die den Operationsort abbildet und somit einem Operateur eine visuelle Inspektion des Operationsorts gestattet.

Bei mikroinvasiv durchgeführten Eingriffen, beispielsweise am Abdomen eines Patienten, benötigt ein Operateur in der Regel beide Hände, um Instrumente zu führen und zu bedienen und auf diese Weise den Eingriff durchzuführen. Ein Endoskop zur optischen Erfassung und Inspektion des Operationsorts wird bisher meist von einem Kameraassistenten gehalten, der z.B. hinter oder neben dem Operateur steht. Dies kann nachteilig sein, weil der Bewegungsraum des Operateurs eingeschränkt ist. Außerdem muss der Kameraassistent oft über einen längeren Zeitraum eine ergonomisch unvorteilhafte Körperhaltung einnehmen, so dass es mit zunehmender Operationsdauer aufgrund Ermüdung des Operationsassistenten zu einem Verwackeln des Bildes kommen kann.

Um einen Kameraassistenten überflüssig zu machen, werden heutzutage auch Haltesysteme eingesetzt, mit denen ein Endoskop statisch an einem Operationstisch montiert werden kann. Solche Haltesysteme verwenden Haltearme, die einen relativ einfachen Aufbau aufweisen und universell einsetzbar sind, eine Veränderung der Lage des Endoskops aber nur manuell durch Verstellen des Haltearms ermöglichen.

Anstelle solcher Haltesysteme sind auch mechanische, insbesondere mechatronische, Führungssysteme bekannt, die eine motorische Verstellung der Lage eines Endoskops ermöglichen. Ein aus der DE 196 09 034 A1 bekanntes Führungssystem ermöglicht beispielsweise, ein Endoskop sowohl um einen Pivotpunkt, der insbesondere einem Einstechpünkt an einer Bauchdecke eines Patienten oder einer anderen Zugangsöffnung an einem Patienten entspricht, zu schwenken, als auch um seine Längsachse zu rotieren.

Dies wird bei dem Führungssystem der DE 196 09 034 A1 dadurch ermöglicht, dass an einem Gehäuse des Endoskops ein Adapter angebracht wird, der über ein Stirnrad mit einem weiteren Stirnrad einer Antriebsvorrichtung derart gekoppelt ist, dass das Endoskop angetrieben durch die Antriebsvorrichtung in eine Drehbewegung versetzt werden kann. Eine Kameravorrichtung, die Licht aus dem Endoskop empfängt, wird hierbei ortsfest gehalten, indem die Kameravorrichtung über einen Haltebügel an einer die Antriebsvorrichtung haltenden Halterung angeordnet ist. Das Endoskop wird also relativ zu der Kameravorrichtung gedreht.

Das Führungssystem gemäß DE 196 09 034 A1 ermöglicht eine Rotation des Endoskops um seine Längsachse und eine entsprechende Einstellung des Sichtfelds. Beispielsweise bei einem Endoskop, das über eine geeignete Optik (sogenannte "Seitenblickoptik") Licht aus einem Bereich seitlich des Endoskopschafts empfängt, dessen feststehende oder einstellbare Blickrichtung nicht parallel zur Längsachse des Endoskopschafts ist, kann durch Rotation des Endoskops um seine Längsachse das Blickfeld geschwenkt und ein Operationsort in unterschiedlichen Betrachtungsrichtungen betrachtet werden.

Dadurch, dass gemäß DE 196 09 034 A1 ein Adapter am Schaft des Endoskops angebracht werden muss, verkürzt sich jedoch die nutzbare Länge des Endoskopschafts, die in einen Patienten eingebracht werden kann. Zudem kommt der Adapter mit dem für eine Operation steril zu haltenden Endoskopschaft in Kontakt, so dass auch Halterungsteile des Führungssystems für eine Operation sterilisiert werden müssen (die Sterilisation erfolgt herkömmlich mittels Autoklavieren in einem Autoklaven).Außerdem können Endoskope unterschiedlicher Hersteller und auch Kameravorrichtungen, die an einem Endoskop zum Einsatz kommen können, in ihrer Bauweise stark voneinander abweichen. Sowohl die Anordnung des Endoskops als auch der Kameravorrichtung an dem Führungssystem kann somit unter Umständen nicht universell für unterschiedliche Endoskope und Kameravorrichtungen gewährleistet werden.

In US 2011/0288374 A1 ist eine endoskopische Vorrichtung zum Abbilden einer inneren Oberfläche eines Hohlraums beschrieben. Zum Rotieren eines endoskopischen Katheters 2 mit einer abgedichteten Kapsel 4, die eine Kamera 6 umfasst, ist eine Antriebseinheit 26 vorgesehen. Die Antriebseinheit 26 wird mittels einer elektrischen Steckverbindung 24 mit dem endoskopischen Katheter verbunden.

In US 2010/0185212 A1 ist ein System zum Positionieren eines Endoskops 7 und eines chirurgischen Instruments beschrieben. Ein Endoskop 7 wird mittels einer Einrichtung 1240 um seine Längsachse rotiert. Eine mit dem Endoskop 7 unmittelbar gekoppelte Kamera 8 wird mittels einer Einrichtung 1270 um ihre Längsachse rotiert.

In US 2012/0065470 A1 ist ein robotisches System für ein Endoskop beschrieben. Das von einer Endoskop-Greif-Anordnung 204 gehaltene Endoskop 100 wird von einer Rotationsanordnung 206 um seine Längsachse rotiert.

Eine Aufgabe der vorliegenden Erfindung ist es, eine verbesserte Rotationsvorrichtung, eine verbesserte Endoskopvorrichtung sowie ein verbessertes Rotieren eines Endoskops bereitzustellen, die insbesondere auf einfache, möglichst universell einsetzbare Weise ein Rotieren des Endoskops um seine Längsachse ermöglichen, um das Sichtfeld zum Inspizieren eines Operationsorts verändern zu können.

Diese Aufgabe wird durch die Gegenstände der unabhängigen Ansprüche gelöst.

Ausführungsformen der vorliegenden Erfindung beruhen auf der Idee, bei einer Rotationsvorrichtung eine an einer feststehenden Baugruppe angeordnete proximale Kopplungseinrichtung zur Kopplung mit einer Kameravorrichtung, eine distale Kopplungseinrichtung an einer rotierbaren Baugruppe und eine Optik zur Übertragung von Licht von der rotierbaren distalen Kopplungseinrichtung zu der proximalen Kopplungseinrichtung vorzusehen.

Dabei weist vorzugsweise entweder die feststehende Baugruppe oder die rotierbare Baugruppe eine Antriebseinrichtung zur Rotation der rotierbaren Baugruppe relativ zur feststehenden Baugruppe auf. Die Antriebseinrichtung umfasst insbesondere einen Elektromotor, einen Ultraschallmotor, einen Piezomotor oder einen anderen Motor.

Ausführungsformen der vorliegenden Erfindung gehen von dem Gedanken aus, eine Rotationsvorrichtung zu schaffen, die nach Art einer modularen Baueinheit zwischen einem Endoskop und einer mit dem Endoskop zu koppelnden, Licht aus dem Endoskop erfassenden Kameravorrichtung angeordnet ist. Dazu weist die Rotationsvorrichtung eine distale Kopplungseinrichtung, die zur vorzugsweise zerstörungsfrei lösbaren mechanischen Verbindung mit dem Endoskop ausgebildet ist, und eine proximale Kopplungseinrichtung, die zur vorzugsweise ebenfalls zerstörungsfrei lösbaren mechanischen Verbindung mit der Kameravorrichtung ausgebildet ist, auf, so dass die Rotationsvorrichtung in einfacher Weise einerseits mit dem Endoskop und andererseits mit der Kameravorrichtung verbunden werden kann. Durch die Optik wird Licht von der distalen Kopplungseinrichtung, also von dem Endoskop, hin zu der proximalen Kopplungseinrichtung, also hin zur Kameravorrichtung, übertragen. An der distalen Kopplungseinrichtung tritt Licht aus dem Endoskop in die Rotationsvorrichtung ein, wird mittels der Optik übertragen und verlässt an der proximalen Kopplungseinrichtung die Rotationsvorrichtung wieder, um der Kameravorrichtung zugeführt zu werden.

Die Ausgestaltung der Rotationsvorrichtung als zwischengeschaltete, zwischen dem Endoskop und der Kameravorrichtung angeordnete modulare Baueinheit wird insbesondere durch die in die Rotationsvorrichtung integrierten Optik ermöglicht. Die Optik dient hierbei zur Übertragung von Licht aus dem Endoskop hin zur Kameravorrichtung und kann Achromaten, Apochromaten, Stablinsen und andere Linsen, Prismen oder andere lichtformende, insbesondere lichtbrechende, Elemente aufweisen, kann aber auch lediglich aus einem oder mehreren planen oder gekrümmten Deckgläsern bestehen, durch die Licht durch die Rotationsvorrichtung hindurch tritt.

Die distale Kopplungseinrichtung ist insbesondere zum Halten eines proximalen Endes eines Endoskops ausgebildet. Die distale Kopplungseinrichtung ist beispielsweise dazu ausgestaltet, einen Okulartrichter eines Endoskops zu halten. Ein solcher Okulartrichter ist insbesondere der deutschen Industrienorm DIN 58105 entsprechend ausgestaltet und weist einen Außendurchmesser von ca. 31,75 mm und einen Rand mit einer Dicke von ca. 4,7 mm auf. Der Okulartrichter kann beispielsweise klemmend, kraft- bzw. reibschlüssig oder formschlüssig an der distalen Kopplungseinrichtung mittels Halteklauen gehalten werden. Die Halteklauen werden in einem Eingriffszustand in Eingriff mit dem Rand des Okulartrichters gebracht, um das Endoskop klemmend, kraft-bzw. reibschlüssig oder formschlüssig an der Rotationsvorrichtung zu halten. Zum Lösen des Endoskops von der Rotationsvorrichtung können die Halteklauen außer Eingriff von dem okularseitigen Ende des Endoskops gebracht werden, um den haltenden Eingriff mit dem Okulartrichter aufzuheben. Alternativ kann die distale Kopplungseinrichtung ausgebildet sein, um statt des Okulars in anderer Weise das proximale Ende des Endoskops kraft- bzw. reibschlüssig oder formschlüssig lösbar zu halten.

Dadurch, dass die distale Kopplungseinrichtung zur Aufnahme und Befestigung des proximalen Endes eines Endoskops, insbesondere des Okulartrichters eines Endoskops, ausgestaltet ist, ergibt sich zum einen, dass die Rotationsvorrichtung an dem das Okular tragenden Ende des Endoskops und somit am proximalen Ende des Endoskops angreift. Es steht somit die gesamte Länge des Endoskopschafts zur Verfügung, um das Endoskop hin zu einem Operationsort zu führen. Zum zweiten verwenden Endoskope in der Regel genormte Okulartrichter, so dass mittels der distalen Kopplungseinrichtung eine Verbindung der Rotationsvorrichtung mit einer Vielzahl von unterschiedlichen Endoskopen möglich und somit eine universelle Anschlussmöglichkeit für unterschiedliche Endoskope geschaffen wird.

Die Verbindung der distalen Kopplungseinrichtung mit dem Okulartrichter des Endoskops ist insbesondere schlupffrei bzw. zur Übertragung eines Drehmoments geeignet ausgebildet, so dass bei einer Drehbewegung der distalen Kopplungseinrichtung das Endoskop schlupffrei mitbewegt wird.

Die rotierbare Baugruppe ist vorzugsweise um eine Längsachse eines mit der Rotationsvorrichtung in bestimmungsgemäßen Zustand verbundenen Endoskops drehbar an der feststehenden Baugruppe gelagert. Das Endoskop kann beispielsweise einen starren Schaft aufweisen, der sich längs erstreckt und eine Längsachse vorgibt. Im Fall eines Endoskops mit einem flexiblen Schaft ist mit der Längsachse insbesondere die Längsachse des proximalen Endes des Schafts gemeint. Das Endoskop ist mittels der distalen Kopplungseinrichtung so mit der Rotationsvorrichtung verbindbar, dass angetrieben durch die Antriebseinrichtung die rotierbare Baugruppe um die Längsachse rotiert und damit auch das Endoskop um seine Längeachse gedreht werden kann, um auf diese Weise die Rotationsposition des Endoskops um die Längsachse zu verstellen.

Die Optik überträgt vorzugsweise Licht, das mittels eines Endoskops in die Rotationsvorrichtung eingeleitet wird, entlang der Längsachse von der distalen Kopplungseinrichtung zu der proximalen Kopplungseinrichtung, so dass an der proximalen Kopplungseinrichtung das Licht austreten und in eine mit der proximalen Kopplungseinrichtung gekoppelte Kameravorrichtung eintreten kann.

Die Optik kann beispielsweise eine erste Linse und eine entlang der Längsachse zu der ersten Linse versetzte zweite Linse aufweisen. Insbesondere weist die Optik einen ersten Achromaten, eine erste Linse, eine zweite Linse und einen zweiten Achromaten auf, die in dieser Reihenfolge entlang der Längsachse zueinander versetzt im optischen Strahlengang zwischen der distalen Kopplungseinrichtung und der proximalen Kopplungseinrichtung angeordnet sind. Licht, das aus einem Endoskop an der distalen Kopplungseinrichtung in die Optik eintritt, durchläuft somit zunächst den ersten Achromaten, dann die erste Linse, dann die zweite Linse und schließlich den zweiten Achromaten, um anschließend an der proximalen Kopplungseinrichtung in die Kameravorrichtung eingeleitet zu werden. Die erste Linse und die zweite Linse können jeweils beispielsweise kurze Linsen, Stablinsen und/oder ihrerseits achromatisch ausgebildet sein. Vor dem ersten Achromaten und hinter dem zweiten Achromaten (betrachtet in Richtung des Strahlengangs) kann zusätzlich jeweils ein Deckglas vorgesehen sein, um die Optik und darin angeordnete Linsen und Achromaten gegen Schmutz und Feuchtigkeit zu schützen. Alternativ können der erste Achromat und/oder der zweite Achromat ausgebildet sein, um die Aufgabe eines Deckglases zu erfüllen.

Alternativ ist eine Ausgestaltung mit anderen Linsensystemen möglich, z.B. mit vier Achromaten und/oder vier nicht-achromatischen Linsen. Ferner kann eine andere Anzahl von Achromaten oder anderen Linsen vorgesehen sein.

Ein Achromat umfasst insbesondere eine Kombination von zwei oder mehr Linsen aus Gläsern oder anderen transparenten Materialien, deren Brechungsindizes in unterschiedlicher Weise von der Wellenlänge abhängen. Die zwei oder mehr Linsen sind so ausgestaltet, dass die Änderung der Schnittweite mit der Wellenlänge, die bei den einzelnen Linsen auftritt, gerade ausgeglichen wird, so dass die für den gesamten Achromaten die Änderung der Schnittweite mit der Wellenlänge für (zumindest) eine Wellenlänge verschwindet. Ein Achromat im Sinne dieser Erfindung umfasst auch einen Apochromaten.

Die Optik kann beispielsweise so ausgestaltet sein, dass sie an oder nahe der proximalen Kopplungseinrichtung ein optisches Bild erzeugt, das im Wesentlichen dem optischen Bild, das von einem Endoskop an oder nahe der distalen Kopplungseinrichtung bereitgestellt wird, entspricht. Das Bild nahe der distalen Kopplungseinrichtung und das Bild nahe der proximalen Kopplungseinrichtung sind insbesondere virtuelle Bilder. Die Optik kann somit eine 1:1-Abbildung oder eine 1:-1-Abbildung bewirken, also eine Abbildung, die im Wesentlichen keine Veränderung der Bildgröße mit sich bringt.

Alternativ ist die Optik ausgebildet, um eine Vergrößerung oder Verkleinerung des Bildes zu bewirken. Alternativ weist die Optik eine veränderbare Brennweite oder einen veränderbaren Fokus auf. Die Einstellung von Fokus und Brennweite kann rein mechanisch über Bedienräder oder -schieber oder ähnliches erfolgen. Alternativ oder ergänzend können ein oder mehrere (weitere) Elektromotoren oder andere Motoren integriert sein, die eine motorische Einstellung von Brennweite und/oder Fokus ermöglichen.

Die Optik ist vorzugsweise in einer Ausnehmung, z.B. einer Bohrung, eines Schaftabschnitts der feststehenden Baugruppe angeordnet und wird somit bei einer Rotation der rotierbaren Baugruppe nicht mitbewegt. Die rotierbare Baugruppe ist insbesondere um den Schaftabschnitt der feststehenden Baugruppe drehbar gelagert, wobei ein oder mehrere Wälz-, Gleit oder andere Lager vorgesehen sein können, um eine leichtgängige Bewegung der rotierbaren Baugruppe um den Schaftabschnitt der feststehenden Baugruppe zu ermöglichen. Die Optik kann vorzugsweise in der Ausnehmung des Schaftabschnitts der feststehenden Baugruppe gekapselt sein derart, dass Schmutz und Feuchtigkeit nicht in die Optik eindringen können und die Optik somit geschützt ist. Dazu sind insbesondere an beiden Enden der Ausnehmung Deckgläser vorgesehen, die plan oder als Linsen ausgebildet sein können, und die so mit dem Schaftabschnitt durch Löten oder auf andere Art gefügt sind, dass sie die Ausnehmung hermetisch dicht verschließen.

In einer alternativen Ausführungsvariante ist die Optik in oder an der rotierbaren Baugruppe angeordnet, vorzugsweise ebenfalls gekapselt in einer Ausnehmung eines Schaftabschnitts oder eines anderen Bauteils der rotierbaren Baugruppe.

Die proximale Kopplungseinrichtung der Rotationsvorrichtung ist insbesondere als Okulartrichter ausgebildet. Der Okulartrichter ist dabei vorzugsweise ebenfalls entsprechend der deutschen Industrienorm DIN 58105 ausgestaltet und.somit in seiner Bauform genormt.

Bei Ausgestaltung der proximalen Kopplungseinrichtung als Okulartrichter ähnlich dem Okulartrichter eines mit der distalen Kopplungseinrichtung zu verbindenden Endoskops kann die Rotationsvorrichtung als modulare, nachrüstbare Baueinheit zwischen ein Endoskop und eine Kameravorrichtung geschaltet werden, ohne dass hierzu bauliche Veränderungen an dem Endoskop oder der Kameravorrichtung vorgenommen werden müssen. Das Endoskop kann in einfacher Weise mit der distalen Kopplungseinrichtung verbunden werden, indem ein Okulartrichter des Endoskops mittels Halteklauen der distalen Kopplungseinrichtung eingespannt oder auf andere Weise befestigt wird. Die Kameravorrichtung kann in einfacher Weise mit der proximalen Kopplungseinrichtung in Form des Okulartrichters verbunden werden. Dabei kann der Okulartrichter der Rotationsvorrichtung an einer Halteeinrichtung der Kameravorrichtung eingespannt oder auf andere Weise befestigt werden, die herkömmlich dazu ausgestaltet ist, einen Okulartrichter eines Endoskops zu halten. Die Rotationsvorrichtung ist somit in variabler, universeller Weise einsetzbar, ohne dass ein Endoskop oder eine Kameravorrichtung in besonderer Weise angepasst werden muss.

Eine Endoskopvorrichtung weist eine Rotationsvorrichtung nach der vorangehend beschriebenen Art, ein mit der distalen Kopplungseinrichtung der Rotationsvorrichtung verbundenes Endoskop und eine mit der proximalen Kopplungseinrichtung der Rotationsvorrichtung verbundene Kameravorrichtung auf. Vorzugsweise kann das Endoskop hierbei einen Schaft und ein an einem von der Rotationsvorrichtung abgewandten distalen Ende angeordnetes, um eine Schwenkachse schwenkbares Schwenkprisma aufweisen. Die Schwenkachse des Schwenkprismas ist nicht parallel, sondern insbesondere orthogonal zur Längsachse des Endoskops. Das Schwenkprisma kann um seine Schwenkachse, beispielsweise mittels eines in das Endoskop integrierten Motors, geschwenkt werden, um auf diese Weise ein insbesondere seitlich aus dem Schaft des Endoskops herausblickendes Sichtfeld in variabler Weise verändern zu können. Dadurch, dass einerseits das Schwenkprisma geschwenkt und andererseits der Schaft des Endoskops mittels der Rotationsvorrichtung um seine Längsachse rotiert werden kann, kann in vorteilhafter Weise ein Operationsort in einem großen Bereich visuell inspiziert werden.

Zum Steuern der Rotationsvorrichtung und zum Steuern des Schwenkprismas kann beispielsweise eine einheitliche Steuerelektronik vorgesehen sein, mittels derer die rotatorische Stellung des Schafts und die Stellung des Schwenkprismas in variabler Weise eingestellt werden können. Alternativ können für die Rotationsvorrichtung einerseits und das Schwenkprisma andererseits unterschiedliche Steuerungselektroniken bereitgestellt werden.

Die Endoskopvorrichtung mit dem Endoskop, der Rotationsvorrichtung und der Kameravorrichtung kann beispielsweise an einem Operationstisch gehalten werden, indem eine Halterung an der Rotationsvorrichtung angreift und über die Rotationsvorrichtung auch das Endoskop und die Kameravorrichtung hält.

Eine Rotationsvorrichtung der hier beschriebenen Art kann mit einem Führungssystem kombiniert werden, bei dem die Lage eines Endoskops im Raum durch Bewegung eines Haltearms in variabler Weise eingestellt werden kann. Durch Rotieren des Endoskops mittels der Rotationsvorrichtung kann auch bei solchen Führungssystemen, insbesondere bei Verwendung von sogenannten Seitenblickendoskopen, die ein seitlich vom Endoskopschaft gerichtetes Blickfeld aufweisen, eine einfache Einstellung des Sichtfelds erreicht werden. Bei Kombination eines Führungssystems mit einer Rotationsvorrichtung kann durch Verwendung von einer Seitenblickoptik eine Vergrößerung des insgesamt einsehbaren Sichtbereichs gegenüber konventionellen Führungssystemen erreicht werden.

Die Aufgabe wird auch gelöst durch ein Rotieren eines Endoskops mittels einer Rotationsvorrichtung, bei dem ein Endoskop an eine distale Kopplungseinrichtung einer rotierbaren Baugruppe angeschlossen wird und eine Antriebseinrichtung die distale Kopplungseinrichtung zum Rotieren des mit der distalen Kopplungseinrichtung verbundenen Endoskops in eine Drehbewegung versetzt. Hierbei ist vorgesehen, dass an eine proximale Kopplungseinrichtung einer feststehenden Baugruppe eine Kameravorrichtung angeschlossen wird und eine Optik Licht von der distalen Kopplungseinrichtung zu der proximalen Kopplungseinrichtung überträgt.

Dabei kann grundsätzlich die feststehende oder die rotierbaren Baugruppe die Antriebseinrichtung aufweisen.

Die vorangehend für die Rotationsvorrichtung beschriebenen Vorteile und vorteilhaften Ausgestaltungen finden analog auch auf das Verfahren Anwendung.

Der der Erfindung zugrunde liegende Gedanke wird nachfolgend anhand der in den Figuren dargestellten Ausführungsbeispieleräher erläutert. Es zeigen:
- Figur 1: eine schematische Darstellung einer Endoskopvorrichtung;
- Figur 2: eine schematische Darstellung einer weiteren Endoskopvorrichtung;
- Figur 3: eine schematische Schnittdarstellung einer Rotationsvorrichtung zum Rotieren eines mit einer rotierbaren Baugruppe der Rotationsvorrichtung verbundenen Endoskops;
- Figur 4: eine schematische axonometrische Darstellung eines Ausführungsbeispiels einer Rotationsvorrichtung;
- Figur 5A: eine weitere schematische Darstellung der Rotationsvorrichtung aus Figur 4;
- Figur 5B: eine weitere schematische Darstellung der Rotationsvorrichtung aus den Figuren 4 und 5A;
- Figur 5C: eine weitere schematische Darstellung der Rotationsvorrichtung aus den Figuren 4 bis 5B;
- Figur 5D: eine weitere schematische Darstellung der Rotationsvorrichtung aus den Figuren 4 bis 5C;
- Figur 6: eine schematische Schnittdarstellung der Rotationsvorrichtung aus den Figuren 4 bis 5D;
- Figur 7: eine schematische axonometrische Darstellung einer weiteren Rotationsvorrichtung;
- Figur 8A: eine weitere schematische Darstellung der Rotationsvorrichtung aus Figur 7;
- Figur 8B: eine weitere schematische Darstellung der Rotationsvorrichtung aus den Figuren 7 und 8A;
- Figur 8C: eine weitere schematische Darstellung der Rotationsvorrichtung aus den Figuren 7 bis 8B; und
- Figur 9: eine schematische Schnittdarstellung der Rotationsvorrichtung aus den Figuren 7 bis 8C.

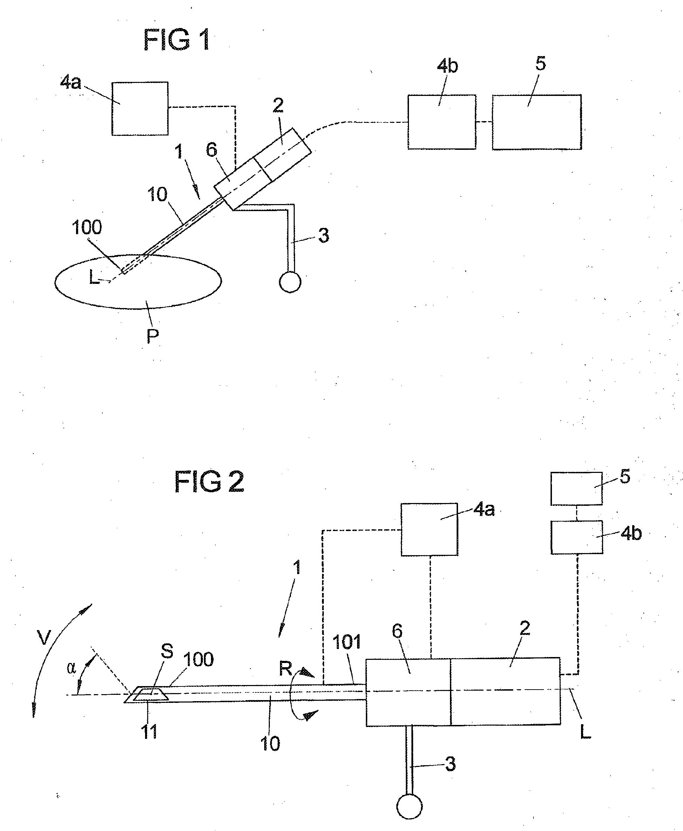

Figur 1 zeigt eine schematische Darstellung einer Endoskopvorrichtung mit einem Endoskop 1 und einer daran angeschlossenen Kameravorrichtung 2, die zur Aufnahme bzw. Erfassung von Bildern aus einem Operationsort an einem Patienten P dient. Das Endoskop 1 weist einen Schaft 10 mit einer Längsachse L auf, dessen distales Ende 100 dem Patienten P zugeführt und in den Patienten P eingeführt ist. Über den Schaft 10 des Endoskops 1 kann Licht von dem Operationsort hin zu der Kameravorrichtung 2 geleitet werden, die das Licht in analoge oder digitale und insbesondere elektrische Signale wandelt und einer Auswerteeinheit 4b zuführt. Die Auswerteeinheit 4b verarbeitet die Signale. Die verarbeiteten Signale werden an eine Anzeigevorrichtung 5 in Form eines Monitors übertragen, um Bilder des Operationsorts anzuzeigen.

Eine Halterung 3 ist beispielsweise an einem Operationstisch befestigt. Die Halterung 3 hält eine Rotationsvorrichtung 6, die das Endoskop 1 mechanisch und optisch mit der Kameravorrichtung 2 koppelt. Die Rotationsvorrichtung 6 ermöglicht eine Rotation des Endoskops 1 um die Längsachse L des Schafts 10 und auf diese Weise eine Variation, insbesondere ein Schwenken, eines Sichtfelds des Endoskops 1.

Zur Steuerung der Rotationsvorrichtung 6 dient eine Steuereinheit 4a. Die Steuereinheit 4a und die Auswerteeinheit 4b können abweichend von der Darstellung in Figur 1 gekoppelt sein, um Signale auszutauschen oder mechanisch und/oder funktionell zu einer Einheit zusammengefasst sein.

Figur 2 zeigt eine schematische Darstellung einer weiteren Endoskopvorrichtung, die in einigen Merkmalen und Eigenschaften der oben anhand der Figur 1 dargestellten Endoskopvorrichtung ähnelt. Nachfolgend sind insbesondere Merkmale und Eigenschaften dargestellt, in denen sich die Endoskopvorrichtung aus Figur 2 von der Endoskopvorrichtung aus Figur 1 unterscheide.

Bei der in Figur 2 dargestellten Endoskopvorrichtung weist das Endoskop 1 an seinem distalen Ende 100 ein Schwenkprisma 11 auf. Das Schwenkprisma 11 ist, beispielsweise mittels eines in das Endoskop integrierten Motors, mittels einer Schub- oder Druckstange oder auf andere Weise, um eine Schwenkachse S schwenkbar. Die Schwenkachse S ist orthogonal zur Längsachse L des Schafts 10 und orthogonal zur Zeichenebene der Figur 2. Mittels des Schwenkprismas 11 kann Licht aus einem insbesondere kegelförmigen, durch einen Sichtwinkel α beschreibbaren Sichtfeld erfasst werden. Durch Schwenken des Schwenkprismas 11 um die Schwenkachse S (Schwenkbewegung V) kann das Sichtfeld variiert, insbesondere in einer Ebene senkrecht zur Schwenkachse S geschwenkt werden. Durch Rotieren des Endoskops 1 mittels der Rotationsvorrichtung 6 kann zusätzlich das Sichtfeld auch um die Längsachse L rotiert werden (Rotationsbewegung R), so dass durch Schwenken des Schwenkprismas 11 einerseits und durch Drehen des Schafts 10 des Endoskops 1 andererseits ein Operationsort in einem großen räumlichen Bereich betrachtet werden kann.

Alternativ zur Verwendung eines Schwenkprismas kann das Schwenken der Blickrichtung auf andere Weise erfolgen, z. B. mittels einer aus Periskopen bekannten Anordnung von reflektierenden Flächen oder durch eine mechanische Abwinklung der Endoskopspitze. Ebenso ist die Verwendung eines Seitenblickendoskops mit definiertem Blickwinkel möglich.

Das proximale Ende 101 des Endoskops 1 ist starr mit der Rotationsvorrichtung 6 verbunden. Die Rotationsvorrichtung 6 wiederum ist mit der Kameravorrichtung 2 mechanisch verbunden. Durch die an der Rotationsvorrichtung 6 angreifende Halterung 3 wird die Endoskopvorrichtung in Position gehalten.

Die Kameravorrichtung 2 ist zum Austausch elektrischer oder anderer Signale und zur Übertragung von Leistung mit der Auswerteeinheit 4b gekoppelt. Die Rotationsvorrichtung 6 und optional das Endoskop 1 sind zum Austausch elektrischer oder anderer Signale und zur Übertragung von Leistung mit der Steuereinheit 4a gekoppelt. Die Auswerteeinheit 4b dient dazu, Signale von der Kameravorrichtung 2 zu empfangen und auszuwerten und der Anzeigevorrichtung 5 zuzuführen. Gleichzeitig kann durch die Steuereinheit 4a die Rotationsvorrichtung 6 und optional auch das Schwenkprisma 11 gesteuert werden, um auf diese Weise die rotatorische Stellung des Schafts 10 sowie die Stellung des Schwenkprismas 11 einzustellen und zu verändern.

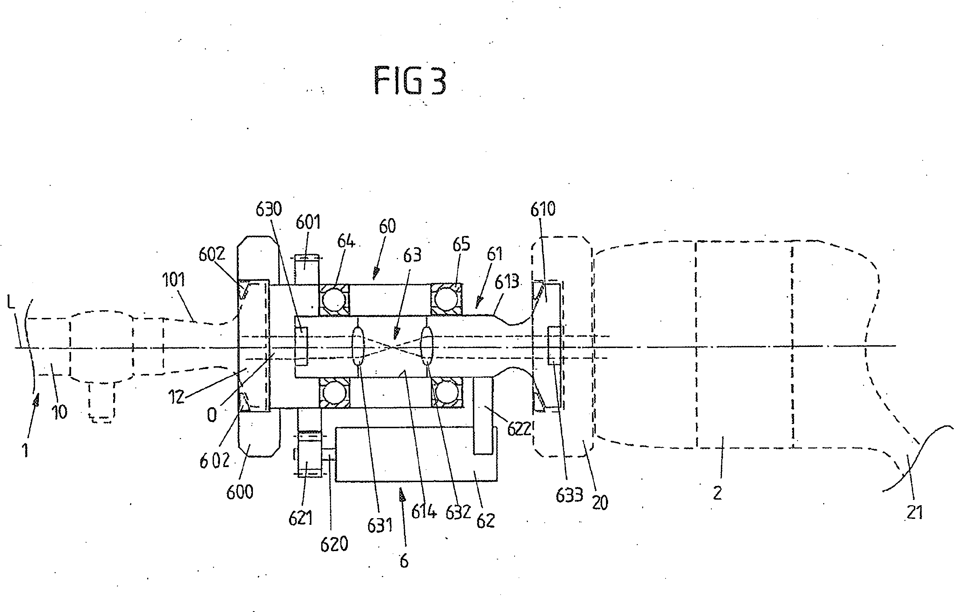

Figur 3 zeigt eine schematische Schnittdarstellung einer Rotationsvorrichtung 6 in durchgezogenen Linien sowie eines Endoskops 1 und einer Kameravorrichtung 2 in gestrichelten Linien. Die Schnittebene der Figur 3 enthält die Längsachse L des Schafts 10 des Endoskops 1. Die Rotationsvorrichtung 6, das Endoskop 1 und die Kameravorrichtung 2 können Merkmale und Eigenschaften aufweisen, die den oben anhand der Figuren 1 und 2 dargestellten ähneln. Insbesondere ist die in Figur 3 dargestellte Rotationsvorrichtung 6 eine der oben anhand der Figuren 1 und 2 dargestellten Rotationsvorrichtungen.

Die Rotationsvorrichtung 6 weist eine feststehende Baugruppe 61 auf, an der über Lager 64, 65 eine rotierbare Baugruppe 60 um die Längsachse L drehbar angeordnet ist. Die Lager 64, 65 sind insbesondere als Kugellager ausgebildet. Die feststehende Baugruppe 61 weist einen Schaftabschnitt 613 auf, an dessen der Kameravorrichtung 2 zugewandtem proximalem Ende eine proximale Kopplungseinrichtung 610 in Form eines Okulartrichter angeordnet ist. Die rotierbare Baugruppe 60 weist eine distale Kopplungseinrichtung 600 auf, die zur mechanischen Kopplung mit einem Okulartrichter 12 des Endoskops 1 ausgebildet ist.

Die proximale Kopplungseinrichtung 610 der feststehenden Baugruppe 61 und der Okulartrichter 12 des Endoskops 1 weisen eine ähnliche oder gleiche Gestalt auf und sind vorzugsweise entsprechend der deutschen Industrienorm 58105 (Stand Juni 2000) ausgestaltet und somit genormt.

Die proximale Kopplungseinrichtung 610 der feststehenden Baugruppe 61 ist mit einer Kopplungseinrichtung 20 der Kameravorrichtung 2 klemmend, kraft- bzw. reibschlüssig oder formschlüssig verbindbar. Die Kopplungseinrichtung 20 der Kameravorrichtung 2 kann ähnlich oder gleich wie die Kopplungseinrichtung 600 der Rotationsvorrichtung 6 aufgebaut sein und z. B. Halteklauen zum klemmenden, kraft- bzw. reibschlüssigen oder formschlüssigen Halten der proximalen Kopplungseinrichtung 610 aufweisen.

Die distale Kopplungseinrichtung 600 der rotierbaren Baugruppe 60 weist Halteklauen 602 auf, mittels derer der Okulartrichter 12 des Endoskops 1 klemmend, kraft- bzw. reibschlüssig oder formschlüssig mit der Rotationsvorrichtung 6 verbunden werden kann. Die Halteklauen 602 stehen dabei in einem Zustand, in dem das Endoskop 1 an der Rotationsvorrichtung 6 angeordnet ist, klemmend, kraft- bzw. reibschlüssig oder formschlüssig mit dem Okulartrichter 12 in Eingriff. Zum Lösen des Endoskops 1 können die Halteklauen 602 außer Eingriff mit dem Okulartrichter 12 gebracht werden, so dass das Endoskop 1 von der Rotationsvorrichtung 6 getrennt werden kann.

Das Endoskop 1 erstreckt sich in einem Zustand, in dem es mit der distalen Kopplungseinrichtung 600 der rotierbaren Baugruppe 60 der Rotationsvorrichtung 6 verbunden ist, entlang der Längsachse L und ist durch Rotieren der rotierbaren Baugruppe 60 um die Längsachse L drehbar. Die Rotationsvorrichtung 6 greift hierbei an dem am proximalen Ende 101 des Schafts 10 angeordneten Okulartrichter 12 und somit ganz am proximalen Ende 101 des Endoskops 1 an, so dass die vollständige Länge des Schafts 10 zum Zuführen des Endoskops 1 hin zu einem Operationsort zur Verfügung steht.

An der feststehenden Baugruppe 61 ist über ein Verbindungsstück 622 eine Antriebseinrichtung 62 in Form eines Elektromotors angeordnet, die über ein an einer Antriebswelle 620 angeordnetes Ritzel 621 mit einem Zahnrad 601 an der rotierbaren Baugruppe 60 in Eingriff steht. Das Zahnrad 601 ist fest an der rotierbaren Baugruppe 60 angeordnet derart, dass angetrieben durch das Ritzel 621 der Antriebseinrichtung 62 das Zahnrad 601 und damit die rotierbare Baugruppe 60 in eine Drehbewegung um die Längsachse L versetzt werden kann, um auf diese Weise die rotatorische Stellung des Endoskops 1 um die Längsachse L zu verändern.

In einer Bohrung 614 des Schaftabschnitts 613 der feststehenden Baugruppe 61 ist eine Optik 63 angeordnet, die - in Richtung des Strahlengangs vom Endoskop 1 hin zur Kameravorrichtung 2 betrachtet - ein erstes Deckglas 630, eine erste Linse 631, eine zweite Linse 632 und ein zweites Deckglas 633 aufweist. Licht O tritt aus dem Endoskop 1 durch das erste Deckglas 630 in die Optik 63 ein, durchläuft die Linsen 631, 632, tritt durch das zweite Deckglas 633 wieder aus der Optik 63 aus und gelangt in die Kameravorrichtung 2.

Die Optik 63 kann beispielsweise eine 1:-1-Abbildung bewirken, insbesondere nahe dem Deckglas 633 ein virtuelles Bild bereitstellen, das einem vom Endoskop 1 nahe dem Deckglas 630 bereitgestellten virtuellen Bild entspricht. Hierunter ist zu verstehen, dass die optische Abbildung in Größe und Form im Wesentlichen nicht verändert ist, also weder vergrößert noch verzerrt, jedoch um 180 Grad rotiert ist.

Durch einen anderen Aufbau der Optik 63 kann ein aufrecht stehendes Bilde bzw. eine Abbildung ohne Seitenumkehr oder Rotation erzeugt werden, z.B. eine 1:1-Abbildung.

Durch die Rotationsvorrichtung 6 wird eine modulare Baueinheit zur Verfügung gestellt, die zwischen dem Endoskop 1 und die Kameravorrichtung 2 geschaltet werden kann, ohne dass hierzu bauliche Veränderungen an dem Endoskop 1 oder der Kameravorrichtung 2 vorgenommen werden müssten. Mit der distalen Kopplungseinrichtung 600 an der rotierbaren Baugruppe 60 und der proximalen Kopplungseinrichtung 610 in Form des Okulartrichters an der feststehenden Baugruppe 61 werden genormte Anschlüsse bereitgestellt, die universell einerseits mit dem Okulartrichter 12 eines Endoskops 1 und andererseits mit einer zur Kopplung mit einem Okulartrichter ausgestalteten Kopplungseinrichtung 20 einer Kameravorrichtung 2 verbunden werden können.

Die Kameravorrichtung 2 weist, wie in Figur 3 dargestellt, einen Anschluss 21 auf, über den beispielsweise zur Übertragung eines Bildsignals eine elektrische oder optische Verbindung mit der Auswerteeinheit 4b hergestellt werden kann.

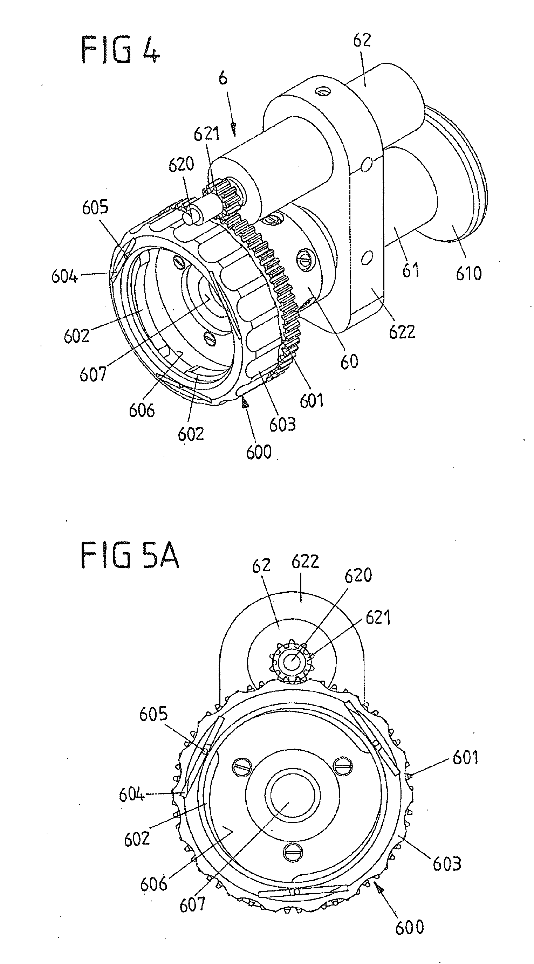

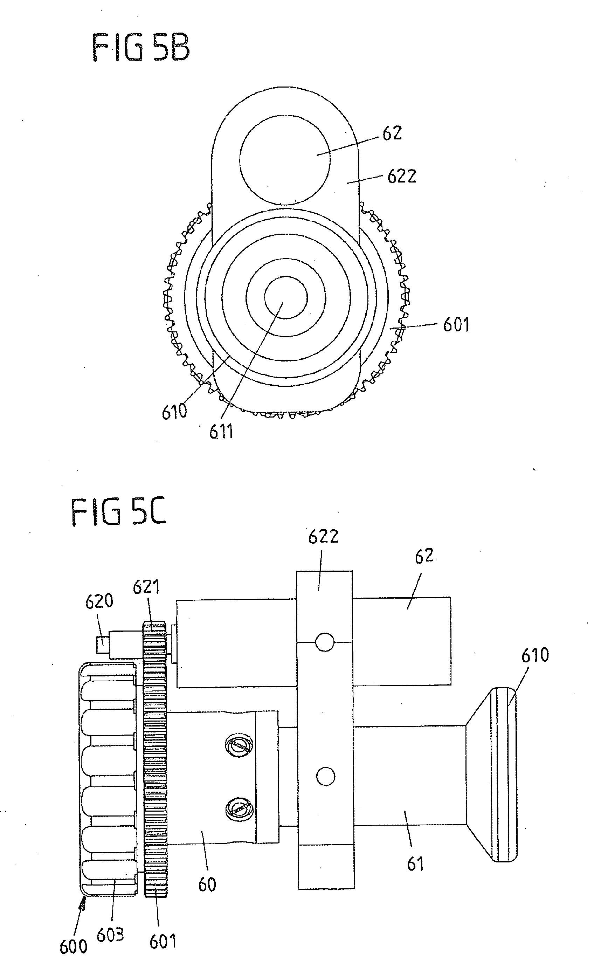

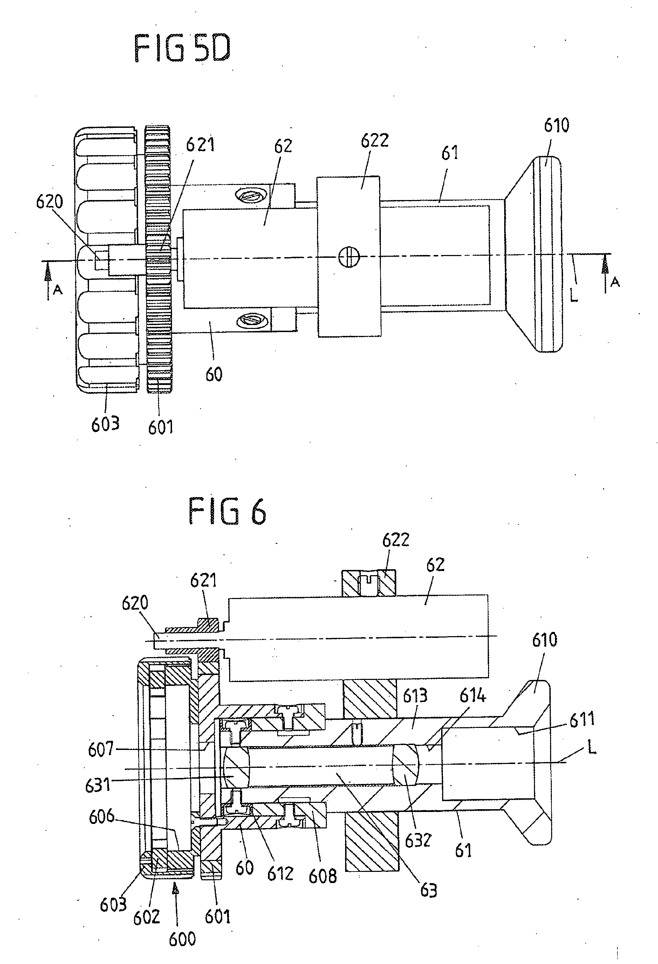

Figuren 4 bis 6 zeigen schematische Darstellungen eines Ausführungsbeispiels einer Rotationsvorrichtung 6, das in einigen Merkmalen und Eigenschaften der oben anhand der Figuren 1 bis 3 dargestellten Rotationsvorrichtung ähnelt. Nachfolgend sind insbesondere Merkmale und Eigenschaften dargestellt, in denen sich die Rotationsvorrichtüng 6 der Figuren 4 bis 6 von der oben anhand der Figuren 1 bis 3 dargestellten unterscheidet. Figur 4 zeigt hierbei eine axonometrische Darstellung der Rotationsvorrichtung 6, Figuren 5A bis 5D zeigen Ansichten von vorne (Figur 5A), von hinten (Figur 5B), von der Seite (Figur 5C) und von oben (Figur 5D). Figur 6 zeigt eine Darstellung eines Schnitts durch die Rotationsvorrichtung 6 entlang der in Figur 5D angedeuteten Ebene A-A.

Die Rotationsvorrichtung 6 weist eine feststehende Baugruppe 61 auf, die über ein Verbindungsstück 622 eine Antriebseinrichtung 62 trägt und beispielsweise an einer Halterung 3 (vgl. Figuren 1 und 2) starr montiert ist. An der feststehenden Baugruppe 61 ist über einen Lagerabschnitt 608 eine rotierbare Baugruppe 60 um eine Längsachse L rotierbar angeordnet. Die rotierbare Baugruppe 60 steht über ein Zahnrad 601 mit einem an einer Antriebswelle 620 der Antriebseinrichtung 62 angeordneten Ritzel 621 kämmend in Eingriff. Der Lagerabschnitt 608 ist an einem Schaftabschnitt 613 in Umfangsrichtung um die Längsachse L gleitend gelagert und über einen Haltering 612 axial an dem Schaft 613 formschlüssig festgelegt, so dass die rotierbare Baugruppe 60 axial an der feststehenden Baugruppe 61 gehalten ist. Der Lagerabschnitt 608 ist insbesondere mittels einer oder mehrere Schrauben mit anderen Teilen der rotierbaren, Baugruppe 60 lösbar verbunden. Der Haltering 612 ist insbesondere mittels einer oder mehrerer Schrauben mit dem Schaftabschnitt 613 lösbar verbunden.

Die rotierbare Baugruppe 60 weist eine distale Kopplungseinrichtung 600 auf, die durch einen Basisteil 606, ein daran angeordnetes Handrad 603 und verstellbar an dem Basisteil 606 angeordnete Halteklauen 602 gebildet ist. Die distale Kopplungseinrichtung 600 dient zur lösbaren mechanischen Kopplung mit einem Okulartrichter 12 eines Endoskops 1 (vgl. z.B. Figuren 1 bis 3). Bei angesetztem Endoskop 1 hält die distale Kopplungseinrichtung 600 mittels der Halteklauen 602 den Okulartrichter 12 klemmend, kraft- bzw. reibschlüssig oder formschlüssig an der rotierbaren Baugruppe 60, so dass das Endoskop 1 zusammen mit der rotierbaren Baugruppe 60 schlupffrei bewegt werden kann.

Jede Halteklauen 602 steht über einen Zapfen 605 mit einer zugeordneten Kulisse 604 an dem Handrad 603 in Eingriff. Dadurch geht eine Drehbewegung des Handrads 603 relativ zu dem Basisteil 606 mit einer zumindest teilweise radialen Bewegung der Halteklauen 602 zwischen radial innen liegenden Positionen in Eingriff mit dem Okulartrichter 12 eines Endoskops 1 und radial außen liegenden Positionen ohne Eingriff einher.

An der feststehenden Baugruppe 61 ist an einem von der rotierbaren Baugruppe 60 abgewandten proximalen Ende des Schaftabschnitts 613 eine proximale Kopplungseinrichtung 610 in Form eines Okulartrichters vorgesehen, die mit einer Kameravorrichtung 2 (vgl. Figuren 1 bis 3) lösbar verbunden werden kann oder durch die ein Nutzer in das Endoskop 1 blicken kann.

In einer sich entlang der Längsachse L erstreckenden und insbesondere zu dieser rotationssymmetrischen Bohrung 614 des Schaftabschnitts 613 der feststehenden Baugruppe 61 ist eine Optik 63 angeordnet, die im Wesentlichen durch Linsen 631, 632 gebildet ist. Durch eine Öffnung 607 an der rotierbaren Baugruppe kann Licht in die Optik 63 eintreten, durch die Optik 63 laufen und an einer Öffnung 611 der feststehenden Baugruppe 61 wieder austreten, um hin zu einer mit der proximalen Kopplungseinrichtung 610 verbundenen Kameravorrichtung 2 geleitet zu werden.

Die Rotationsvorrichtung 6 verwirklicht eine modulare Baueinheit, die über die distale Kopplungseinrichtung 600 mit einem Endoskop 1 und über die proximale Kopplungseinrichtung 610 mit einer Kameravorrichtung 2 verbunden werden kann. Die distale Kopplungseinrichtung 600 ist zur Kopplung mit einem genormten Okulartrichter 12 eines Endoskops 1 ausgestaltet, und die proximale Kopplungseinrichtung 610 weist die Gestalt eines ebenfalls genormten Okulartrichters auf. Deshalb kann die Rotationsvorrichtung 6 universell mit herkömmlichen Endoskopen 1 und Kameravorrichtungen 2 verbunden werden, ohne dass eine Anpassung des Endoskops 1 oder der Kameravorrichtung 2 notwendig wäre.

Mittels der Rotationsvorrichtung 6 kann ein Endoskop 1 um die Längsachse L rotiert werden, wobei das Endoskop 1 sich mit seinem Schaft 10 entlang der Längsachse L erstreckt, wenn es über die erste Kopplungseinrichtung 600 klemmend, kraft- bzw. reibschlüssig oder formschlüssig mit der rotierbaren Baugruppe 60 verbunden ist.

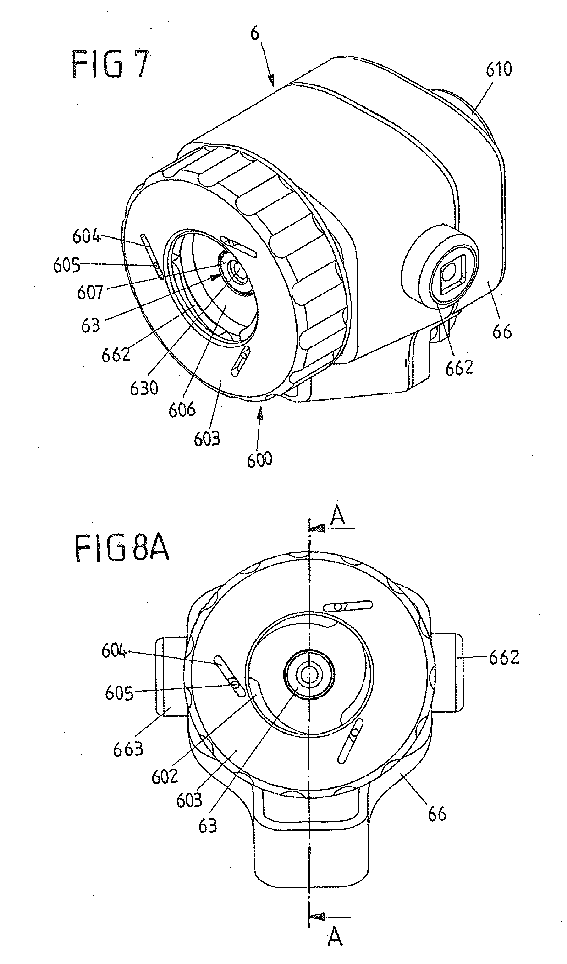

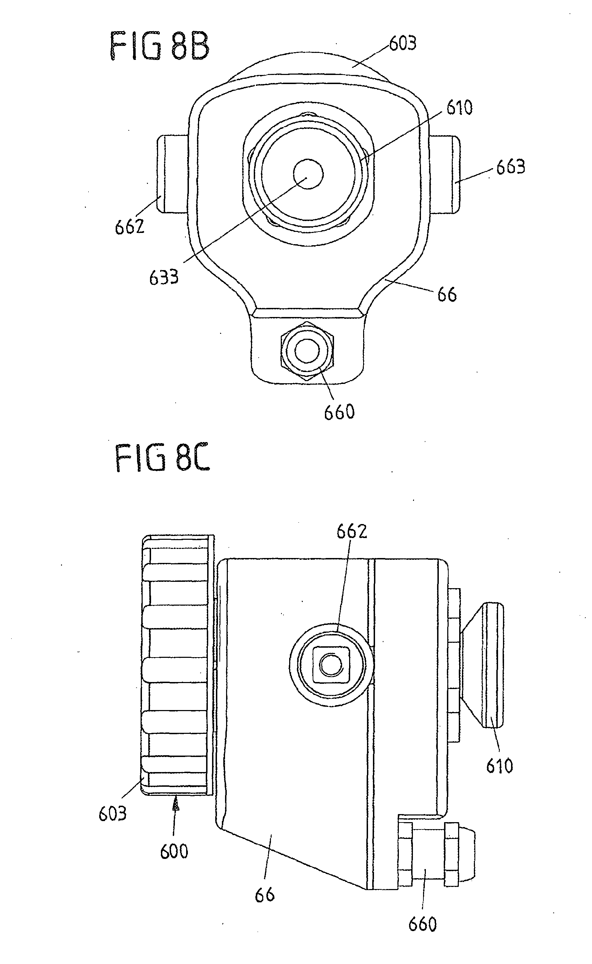

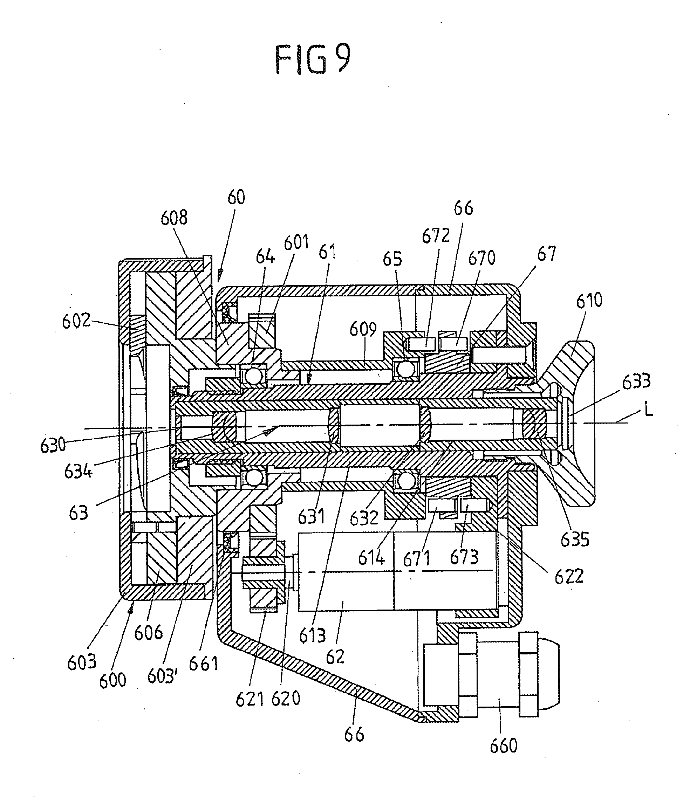

Die Figuren 7 bis 9 zeigen schematische Darstellungen eines Ausführungsbeispiels einer Rotationsvorrichtung 6, das in einigen Merkmalen und Eigenschaften den oben anhand der Figuren 1 bis 6 dargestellten Rotationsvorrichtungen ähnelt. Nachfolgend sind insbesondere Merkmale und Eigenschaften dargestellt, in denen sich die Rotationsvorrichtung 6 der Figuren 7 bis 9 von der oben anhand der Figuren 1 bis 3 dargestellten unterscheidet. Figur 7 zeigt eine axonometrische Ansicht, Figur 8A eine Ansicht von vorn, Figur 8B eine Ansicht von hinten, Figur 8C eine Seitenansicht und Figur 9 eine Darstellung eines Schnitts entlang der in Figur 8A angedeuteten Ebene A-A.

Die in den Figuren 7 bis 9 dargestellte Rotationsvorrichtung 6 weist, ähnlich wie die oben anhand der Figuren 1 bis 6 dargestellten Rotationsvorrichtungen, eine feststehende Baugruppe 61, eine rotierbare Baugruppe 60, eine Optik 63 und eine Antriebseinrichtung 62 auf. Die rotierbare Baugruppe 60 ist mittels zweier Lager 64, 65 rotierbar an der feststehenden Baugruppe 61 gelagert. Die Lager 64, 65 sind zwischen einem Schaftabschnitt 613 der feststehenden Baugruppe 61 und Lagerabschnitten 608, 609 an der rotierbaren Baugruppe 60 angeordnet. An dem Lagerabschnitt 608 ist ein Zahnrad 601 starr angeordnet, das mit einem Ritzel 621 an einer Antriebswelle 620 der Antriebseinrichtung 62 kämmend in Eingriff steht. Dadurch bewirkt eine von der Antriebseinrichtung 62 erzeugte Rotation des Ritzels 621 eine Drehbewegung des Zahnrads 601 und der rotierbaren Baugruppe 60.

Abweichend von der Darstellung anhand der Figuren 7 bis 9 kann eine Antriebseinrichtung für eine erfindungsgemäße Rotationsvorrichtung ein alternativ ausgestaltetes Getriebe aufweisen oder mit diesem gekoppelt sein. Das Getriebe ist beispielsweise ein Schneckengetriebe oder ein Kettengetriebe.

Abweichend von der Darstellung anhand der Figuren 7 bis 9 kann eine Antriebseinrichtung ohne Getriebe vorgesehen sein. Zum Beispiel kann eine solche Antriebseinrichtung einen sogenannten Hohlläufer oder einen Piezo- oder Ultraschallmotor aufweisen, der insbesondere symmetrisch zur Längsachse L bzw. koaxial zum Schaftabschnitt 613 der feststehenden Baugruppe 61, zur Bohrung 614 und zu den Lagerabschnitten 608, 609 angeordnet ist.

Die rotierbare Baugruppe 60 weist eine distale Kopplungseinrichtung 600 auf, die durch ein Basisteil 606, ein Handrad 603 und Halteklauen 602 gebildet ist. Die Halteklauen 602 dienen, ähnlich wie oben anhand der Figuren 4 bis 6 beschrieben, zum klemmenden, kraft- bzw. reibschlüssigen oder formschlüssigen Halten eines Okulartrichters 12 eines Endoskops. Durch Drehung des Handrads 603 relativ zu dem Basisteil 606 können die Halteklauen 602 nach radial innen bewegt und dadurch klemmend, kraft- bzw. reibschlüssig oder formschlüssig in Eingriff mit einem Okulartrichter 12 oder, zum Lösen des Okulartrichters 12, nach radial außen und damit außer Eingriff mit dem Okulartrichter 12 bewegt werden. Das Handrad 603 ist mit einem Haltering 603' fest verbunden und umgreift mit dem Haltering 603' das Basisteil 606, so dass das Handrad 603 nicht axial relativ zum Basisteil 606 bewegbar ist.

Die Optik 63 der feststehenden Baugruppe 61 weist, entlang des Strahlengangs von der distalen Kopplungseinrichtung 600 hin zur proximalen Kopplungseinrichtung 610 betrachtet, ein Deckglas 630, einen ersten Achromaten 634, eine erste einfache Linse 631, eine zweite einfache Linse 632, einen zweiten Achromaten 635 und ein weiteres Deckglas 633 auf. Die Optik 63 leitet Licht aus einem mit der distalen Kopplungseinrichtung 600 verbundenen Endoskop 1 hin zur proximalen Kopplungseinrichtung 610 und zu einer Kameravorrichtung 2, die mit der proximalen Kopplungseinrichtung 610 verbunden ist.

Die Optik 63 bewirkt insbesondere eine 1:1-Abbildung oder eine 1:-1-Abbildung des vom Endoskop 1 empfangenen Bildes im oben anhand der Figur 3 beschriebenen Sinne und verzerrt oder vergrößert das Bild somit nicht.

Die Optik 63 ist in einer Bohrung 614 des Schaftabschnitts 613 angeordnet, die zur Längsachse L rotationssymmetrisch ist. Mittels der Deckgläser 630, 633 ist die Optik 63 in der Bohrung 614 gekapselt, insbesondere hermetisch gekapselt, und somit vor Feuchtigkeit und Schmutz geschützt.

Die anhand der Figuren 7 bis 9 dargestellte Rotationsvorrichtung 6 weist ein Gehäuse 66 auf, das fest mit der feststehenden Baugruppe 61 verbunden ist und über einen Dichtring 661 dichtend an einer äußeren Mantelfläche des Lagerabschnitts 608 anliegt. Das Gehäuse 66 kapselt insbesondere die feststehende Baugruppe 61 mit der daran angeordneten Antriebseinrichtung 62 und schützt diese somit vor Feuchtigkeit und Schmutz.

An dem Gehäuse 66 sind, wie in den Figuren 7 bis 8C dargestellt, Anschlüsse 662, 663 angebracht, über die eine Halterung 3 an der Rotationsvorrichtung 6 befestigt, beispielsweise mit der Rotationsvorrichtung 6 verschraubt werden kann. An dem Gehäuse 66 ist zudem ein Anschluss 660 vorgesehen, über den ein elektrisches Kabel hin zu der Antriebseinrichtung 62 geführt werden kann, um die Antriebseinrichtung 62 elektrisch zu versorgen und zu steuern. Der Anschluss 660 ist insbesondere als gegen Fluide abgedichtete Kabeldurchführung mit Zugentlastung oder als elektrischer und/oder optischer Steckverbinder ausgebildet.

An dem Schaftabschnitt 613 der feststehenden Baugruppe 61 ist weiterhin ein rotierbarer Zwischenring 67 angeordnet, der Anschläge 670, 671 aufweist. Die Anschläge 670, 671 begrenzen zusammen mit einem Anschlag 672 an dem Lagerabschnitt 609 der rotierbaren Baugruppe 60 einerseits und zusammen mit dem Anschlag 673 am Verbindungsstück 622 der feststehenden Baugruppe 61 andererseits den Schwenkwinkel der rotierbaren Baugruppe 60 relativ zu der feststehenden Baugruppe 61. Der Zwischenring 67 ist hierbei selbst drehbar an dem Schaftabschnitt 613 gelagert und kann abschnittsweise zusammen mit der rotierbaren Baugruppe 60 bewegt werden, wenn der Anschlag 672 der rotierbaren Baugruppe 60 mit dem Anschlag 671 des Zwischenrings 67 in Anlage gelangt, bis der Anschlag 670 des Zwischenrings 67 an dem Anschlag 673 der feststehenden Baugruppe anschlägt. Mittels des Zwischenrings 67 kann je nach Anordnung und Gestaltung der Anschläge 670, 671, 672 und 673 der Drehweg der rotierbaren Baugruppe 60 relativ zu der feststehenden Baugruppe 61 auf einen Drehwinkel von ca. 360 Grad oder von ca. 720 Grad oder auf einen größeren oder kleineren Drehwinkel begrenzt werden. Durch die Begrenzung des Drehwegs kann ein Aufwickeln eines Lichtleiters am Endoskop 1 vermieden und/oder die Kalibrierung der Motorposition bzw. der rotatorischen Position der rotierbaren Baugruppe ermöglicht werden.

Auch die anhand der Figuren 7 bis 9 dargestellte Rotationsvorrichtung 6 wird als modulare Baueinheit bereitgestellt, die einerseits mit einem Endoskop 1 und andererseits mit einer Kameravorrichtung 2 verbunden werden kann. Die Rotationsvorrichtung 6 kann in universeller Weise zwischen einem einen genormten Okulartrichter 12 aufweisenden Endoskop 1 und einer zur Verbindung mit einer proximalen Kopplungseinrichtung 610 ausgebildeten Kameravorrichtung 2 angeordnet werden. Deshalb kann die Rotationsvorrichtung 6 ohne weiteres bei bestehenden Endoskopvorrichtungen nachgerüstet werden.

Der der Erfindung zugrunde liegende Gedanke ist nicht auf die vorangehend geschilderten Ausführungsbeispiele beschränkt, sondern lässt sich auch bei gänzlich anders gearteten Ausführungsformen verwirklichen.

Insbesondere ist eine Rotationsvorrichtung der hier beschriebenen Art nicht notwendigerweise beschränkt auf einen medizinischen Einsatz, sondern kann auch bei Endoskopvorrichtungen in anderen Bereichen, beispielsweise im Bereich der technischen Endoskopie zur Materialprüfung oder Fertigungskontrolle eingesetzt werden.

Die Optik der Rotationsvorrichtung muss keine 1:-1-Abbildung oder 1:1-Abbildung sein, sondern kann auch vergrößernd oder verkleinernd wirken. Alternativ kann die Optik dadurch verstellbar ausgegestaltet sein, dass einzelne Linsen entlang der Längsachse bewegbar sind, so dass mittels der Optik der Rotationsvorrichtung gleichzeitig beispielsweise auch eine Zoomstellung oder eine Fokusstellung verändert werden kann. Eine Einstellung der Zoom- oder Fokusstellung kann manuell, beispielsweise über Bedienräder oder -schieber oder ähnliches, und/oder motorisch erfolgen.

Im Allgemeinen benötigt eine Optik mit gespiegelter, insbesondere um 180 Grad rotierter, Abbildung (z.B. 1:-1-Abbildung) weniger Baulänge als eine Optik mit seitenrichtiger Abbildung (z.B. 1:1-Abbildung). Um die Baulänge der Rotationsvorrichtung zu reduzieren, bietet eine Optik mit gespiegelter Abbildung in dieser Hinsicht Vorteile. Außerdem ist die Anzahl an Linsen geringer und dadurch die Übertragungsqualität höher. Um das Bild für die Darstellung am Bildschirm seitenrichtig darzustellen, kann eine entsprechende Umformung der digitalen oder analogen Bildsignale mittels zusätzlicher in der Auswerteeinheit 4b integrierter Hardware und/oder Software realisiert werden. Alternativ wird die Kameravorrichtung 2 um 180 Grad rotiert mit der proximalen Kopplungseinrichtung 610 verbunden.

Um die Auswahl an verwendbaren Auswerteeinheiten nicht einzuschränken, ist es alternativ möglich, zwischen einer konventionellen Auswerteeinheit 4b und der Anzeigevorrichtung 5 eine zusätzliche Hardwarekomponente zu integrieren, die ein von der Auswerteeinheit 4b ausgehendes gespiegeltes Bildsignal seitenrichtig umformt und an die Anzeigevorrichtung 5 weiterleitet. In einer Ausführungsvariante kann diese Hardwarekomponente auch mit der Steuereinheit 4a in einer Einheit mechanisch und/oder funktionell zusammengefasst sein.

Bei den anhand der Figuren 1 bis 9 dargestellten Rotationsvorrichtungen ist die Optik 63 in einer Bohrung 614 bzw. in einem Kanal bzw. in einer Ausnehmung an der feststehenden Baugruppe 61 angeordnet. Alternativ kann die Optik an der rotierbaren Baugruppe angeordnet sein, um mit dieser zu rotieren. Dazu ist insbesondere abweichend von den Darstellungen anhand der Figuren 3 bis 9 die rotierbare Baugruppe zumindest teilweise innerhalb der feststehenden Baugruppe angeordnet.

### Bezugszeichenliste

- 1: Endoskop
- 10: Schaft
- 100: Distales Ende des Endoskops 1
- 101: Proximales Ende des Endoskops 1
- 11: Schwenkprisma am distalen Ende 100 des Endoskops 1
- 12: Okulartrichter am proximalen Ende 101 des Endoskops 1
- 2: Kameravorrichtung
- 20: Kopplungseinrichtung der Kameravorrichtung 2
- 3: Halterung zum Halten der Rotationsvorrichtung 6
- 4a: Steuereinheit zum Steuern der Rotationsvorrichtung 6
- 4b: Auswerteeinheit zum Auswerten eines Bildsignals von der Kameravorrichtung 2 und zum Erzeugen eines Bildsignals für die Anzeigevorrichtung 5
- 5: Anzeigevorrichtung zum Anzeigen eines Bilds gesteuert durch die Auswerteeinheit 4b
- 6: Rotationsvorrichtung zum Rotieren des Endoskops 2
- 60: Rotierbare Baugruppe der Rotationsvorrichtung 6
- 600: distale Kopplungseinrichtung an der rotierbaren Baugruppe 60
- 601: Zahnrad an der rotierbaren Baugruppe 60
- 602: Halteklaue an der distalen Kopplungseinrichtung 600
- 603: Handrad an der distalen Kopplungseinrichtung 600
- 603': Haltering am Handrad 603
- 604: Kulisse für Zapfen 605 im Handrad 603
- 605: Zapfen an Halteklaue 602
- 606: Basisteil der rotierbaren Baugruppe 60
- 607: Öffnung an der rotierbaren Baugruppe 60
- 608, 609: Lagerabschnitt der rotierbaren Baugruppe 60
- 61: Feststehende Baugruppe
- 610: proximale Kopplungseinrichtung an der feststehenden Baugruppe 61
- 611: Öffnung am proximalen Ende der feststehenden Baugruppe 61
- 612: Haltering an der feststehenden Baugruppe 61
- 613: Schaftabschnitt der feststehenden Baugruppe 61
- 614: Bohrung (Ausnehmung) im Schaftabschnitt 613
- 62: Antriebseinrichtung zum Rotieren der rotierbaren Baugruppe 60 relativ zur feststehenden Baugruppe 61
- 620: Antriebswelle der Antriebseinrichtung 62
- 621: Ritzel an der Antriebswelle 620 der Antriebseinrichtung 62
- 622: Verbindungsstück zum Halten der Antriebseinrichtung 62 an der feststehenden Baugruppe 61
- 63: Optik in der Bohrung 614 der feststehenden Baugruppe 61
- 630: Deckglas am distalen Ende der Optik 63 bzw. der Bohrung 614
- 631, 632: Linse der Optik 63
- 633: Deckglas am proximalen Ende der Optik 63 bzw. der Bohrung 614
- 634, 635: Achromat der Optik 63
- 64, 65: Lager zwischen der rotierbaren Baugruppe 60 und der feststehenden Baugruppe 61
- 66: Gehäuse der Rotationsvorrichtung 6
- 660: Anschluss am Gehäuse 66
- 661: Dichtring zwischen dem Gehäuse 66 und der rotierbaren Baugruppe 60
- 662, 663: Anschlussstutzen am Gehäuse 66
- 67: Zwischenring zur Begrenzung der Rotation der rotierbaren Baugruppe 60 relativ zur feststehenden Baugruppe 61
- 670, 671: Anschlag am Zwischenring 67
- 672: Anschlag an der rotierbaren Baugruppe 60, korrespondierend zum Anschlag 671 am Zwischenring 67
- 673: Anschlag an der feststehenden Baugruppe 61, korrespondierend zum Anschlag 670 am Zwischenring 67
- α: Sichtwinkel
- L: Längsachse des Endoskops 1 und optische Achse der Optik 63 der Rotationsvorrichtung 6 und Rotationsachse der rotierbaren Baugruppe 60
- O: Licht
- P: Patient
- R: Rotationsbewegung um die Längsachse L
- S: Schwenkachse des Schwenkprismas 11
- V: Schwenkbewegung der Blickrichtung des Endoskops 1

## Patentansprüche

1. Rotationsvorrichtung (6) zum Rotieren eines Endoskops (1), mit
einer feststehenden Baugruppe (61);
einer rotierbaren Baugruppe (60);
einer distalen Kopplungseinrichtung (600) an der rotierbaren Baugruppe (60) zur Kopplung mit einem Endoskop(1);
einer Antriebseinrichtung, die ausgebildet ist, die distale Kopplungseinrichtung (600) in eine Drehbewegung (R) zu versetzen, um ein mit der distalen Kopplungseinrichtung (600) gekoppeltes Endoskop (1) zu rotieren; **gekennzeichnet durch**
einer proximalen Kopplungseinrichtung (610) an der feststehenden Baugruppe (61) zur Kopplung mit einer Kameravorrichtung (2); und
einer Optik (63) zum Übertragen von Licht von der distale Kopplungseinrichtung (600) zu der proximalen Kopplungseinrichtung (610).

2. Rotationsvorrichtung (6) nach dem vorangehenden Anspruch, bei der die erste Kopplungseinrichtung (600) ausgebildet ist, eine lösbare mechanische Verbindung mit einem Endoskop (1) herzustellen.

3. Rotationsvorrichtung (6) nach einem der vorangehenden Ansprüche, bei der die erste Kopplungseinrichtung (600) ausgebildet ist, einen Okulartrichter (12) des Endoskops (1) oder in anderer Weise ein proximales Ende (101) des Endoskops zu halten.

4. Rotationsvorrichtung (6) nach einem der vorangehenden Ansprüche, bei der die rotierbare Baugruppe (60) um eine Längsachse (L) eines mit der Rotationsvorrichtung (6) in bestimmungsgemäßem Zustand verbundenen Endoskops (1) drehbar an der feststehenden Baugruppe (61) gelagert ist.

5. Rotationsvorrichtung (6) nach Anspruch 4, bei der die Optik (63) ausgebildet ist, um Licht entlang der Längsachse (L) von der distalen Kopplungseinrichtung (600) zu der proximalen Kopplungseinrichtung (610) zu übertragen.

6. Rotationsvorrichtung (6) nach Anspruch 4 oder 5, bei der die Optik (63) eine erste Linse (631) und eine entlang der Längsache (L) zu der ersten Linse (631) versetze, zweite Linse (632) aufweist.

7. Rotationsvorrichtung (6) nach einem der Ansprüche 4 bis 6, bei der die Optik (63) einen ersten Achromaten (634), eine erste Linse (631), eine zweite Linse (632) und einen zweiten Achromaten (635) aufweist, die in dieser Reihenfolge entlang der Längsachse (L) zueinander versetzt im optischen Strahlengang zwischen der distalen Kopplungseinrichtung (600) und der proximalen Kopplungseinrichtung (610) angeordnet sind.

8. Rotationsvorrichtung (6) nach einem der vorangehenden Ansprüche, bei der die Optik (63) ausgebildet ist um nahe der proximalen Kopplungseinrichtung (610) ein optisches Bild zu erzeugen, das im Wesentlichen einem optischen Bild, das von einem mit der Rotationsvorrichtung gekoppelten Endoskop (1) nahe der distalen Kopplungseinrichtung (600) bereitgestellt wird, entspricht.

9. Rotationsvorrichtung (6) nach einem der vorangehenden Ansprüche, bei der die Optik (63) in einer Ausnehmung (614) eines Schaftabschnitt (613) der feststehenden Baugruppe (61) angeordnet und die rotierbare Baugruppe (60) um den Schaftabschnitt (613) drehbar gelagert ist.

10. Rotationsvorrichtung (6) nach einem der vorangehenden Ansprüche, bei der das die proximale Kopplungseinrichtung (610) als Okulartrichter ausgebildet ist.

11. Rotationsvorrichtung (6) nach Anspruch 10, bei der die als Okulartrichter ausgebildete proximale Kopplungseinrichtung (610) so ausgebildet ist wie ein Okulartrichter (12) eines mit der distalen Kopplungseinrichtung (600) zu verbindenden Endoskops (1).

12. Rotationsvorrichtung (6) nach einem der vorangehenden Ansprüche, bei der die Antriebseinrichtung (62) ein Ritzel (621) aufweist, das mit einem Zahnrad (601) der rotierbaren Baugruppe (60) in Eingriff steht.

13. Endoskopvorrichtung mit:
einer Rotationsvorrichtung (6) nach einem der vorangehenden Ansprüche;
einem mit der distalen Kopplungseinrichtung (600) der Rotationsvorrichtung (6) verbindbaren Endoskop (1);
einer mit der proximalen Kopplungseinrichtung (610) der Rotationsvorrichtung (6) verbindbaren Kameravorrichtung (2).

14. Endoskopvorrichtung nach Anspruch 13, bei der das Endoskop (1) einen Schaft (10) und ein an einem von der Rotationsvorrichtung (6) abgewandten Ende (100) angeordnetes, um eine Schwenkachse (S) schwenkbares Schwenkprisma (11) aufweist.

## Claims

1. Rotational device (6) for rotating an endoscope (1) comprising
a stationary assembly (61);
a rotatable assembly (60);
a distal coupling means (600) at the rotatable assembly (60) for coupling to an endoscope (1);
a drive means, which is embodied for putting the distal coupling means (600) into rotational motion (R) in order to rotate an endoscope (1) coupled to the distal coupling means (600); **characterized by**
a proximal coupling means (610) at the stationary assembly (61) for coupling to a camera device (2); and
an optical unit (63) for transmitting light from the distal coupling means (600) to the proximal coupling means (610).

2. Rotational device (6) according to the preceding claim, wherein the first coupling means (600) is embodied for establishing a detachable mechanical connection to an endoscope (1).

3. Rotational device (6) according to one of the preceding claims, wherein the first coupling means (600) is embodied for holding an eyepiece (12) of the endoscope (1) or for holding a proximal end (101) of the endoscope in a different way.

4. Rotational device (6) according to one of the preceding claims, wherein the rotatable assembly (60) is mounted at the stationary assembly (61) in a manner rotatable about a longitudinal axis (L) of an endoscope (1) connected to the rotational device (6) in the intended state.

5. Rotational device (6) according to Claim 4, wherein the optical unit (63) is embodied for transmitting light from the distal coupling means (600) to the proximal coupling means (610) along the longitudinal axis (L).

6. Rotational device (6) according to Claim 4 or 5, wherein the optical unit (63) comprises a first lens (631) and a second lens (632) offset along the longitudinal axis (L) in relation to the first lens (631).

7. Rotational device (6) according to one of Claims 4 to 6, wherein the optical unit (63) comprises a first achromat (634), a first lens (631), a second lens (632) and a second achromat (635) which are arranged in this sequence along the longitudinal axis (L), offset with respect to one another, in the optical beam path between the distal coupling means (600) and the proximal coupling means (610).

8. Rotational device (6) according to one of the preceding claims, wherein the optical unit (603) is embodied for generating an optical image in the vicinity of the proximal coupling means (610), which image substantially corresponds to an optical image that is provided in the vicinity of the distal coupling means (600) by an endoscope (1) coupled to the rotational device.

9. Rotational device (6) according to one of the preceding claims, wherein the optical unit (603) is arranged in a cutout (614) of a shaft section (613) of the stationary assembly (61) and the rotatable assembly (60) is rotatably mounted about the shaft section (613).

10. Rotational device (6) according to one of the preceding claims, wherein the proximal coupling means (610) is embodied as an eyepiece.

11. Rotational device (6) according to Claim 10, wherein the proximal coupling means (610) embodied as an eyepiece is embodied like an eyepiece (12) of an endoscope (1) to be connected to the distal coupling means (600).

12. Rotational device (6) according to one of the preceding claims, wherein the drive device (62) comprises a pinion (621) which engages with a gearwheel (601) of the rotatable assembly (60).

13. Endoscope device comprising:
a rotational device (6) according to one of the preceding claims;
an endoscope (1) connectable to the distal coupling means (600) of the rotational device (6);
a camera device (2) connectable to the proximal coupling means (610) of the rotational device (6).

14. Endoscope device according to Claim 13, wherein the endoscope (1) comprises a shaft (10) and a swivel prism (11), swivelable about a swivel axis (S) and arranged at an end (100) distant from the rotational device (6).

## Revendications

1. Dispositif de rotation (6) pour faire tourner un endoscope (1) avec :
un ensemble fixe (61) ;
un ensemble pivotant (60) ;
un dispositif de couplage distal (600) prévu au ensemble pivotant (60) pour le couplage avec un endoscope (1) ;
un dispositif d'entraînement réalisé pour déplacer le dispositif de couplage distal (600) dans un mouvement de rotation (R), pour faire tourner un endoscope (1) couplé au dispositif de couplage distal (600) ; **caractérisé par** :
un dispositif de couplage proximal (610) prévu au ensemble fixe (61) pour le couplage avec un dispositif de roue de caméra (2) ; et
un système optique (63) prévu pour transmettre la lumière du dispositif de couplage distal (600) au dispositif de couplage proximal (610).

2. Dispositif de rotation (6) selon la revendication précédente, dans lequel le premier dispositif de couplage (600) est réalisé pour établir une liaison mécanique amovible avec un endoscope (1).

3. Dispositif de rotation (6) selon l'une quelconque des revendications précédentes, dans lequel le premier dispositif de couplage (600) est réalisé pour maintenir un entonnoir oculaire (12) de l'endoscope (1) ou autrement une extrémité proximale (101) de l'endoscope.

4. Dispositif de rotation (6) selon l'une quelconque des revendications précédentes, dans lequel le ensemble pivotant (60) est disposé autour d'un axe longitudinal (L) d'un endoscope (1) relié au dispositif de rotation (6) à l'état conforme à la disposition de façon à pouvoir pivoter au ensemble fixe (61).

5. Dispositif de rotation (6) selon la revendication 4, dans lequel le système optique (63) est réalisé pour transmettre la lumière le long de l'axe longitudinal (L) du dispositif de couplage distal (600) au dispositif de couplage proximal (610).

6. Dispositif de rotation (6) selon la revendication 4 ou 5, dans lequel le système optique (63) comporte une première lentille (631) et une deuxième lentille (632) décalée par rapport à la première lentille (631) le long de l'axe longitudinal (L).

7. Dispositif de rotation (6) selon l'une quelconque des revendications 4 à 6, dans lequel le système optique (63) comporte un premier achromate (634), une première lentille (631), une deuxième lentille (632) et un deuxième achromate (635) disposés les uns par rapport aux autres dans cet ordre le long de l'axe longitudinal (L) de façon à être décalés dans le couloir de rayon optique entre le dispositif de couplage distal (600) et le dispositif de couplage proximal (610).

8. Dispositif de rotation (6) selon l'une quelconque des revendications précédentes, dans lequel le système optique (63) est réalisé pour produire à proximité du dispositif de couplage proximal (610) une image optique correspondant pour l'essentiel à une image optique mise à disposition par un endoscope (1) couplé au dispositif de rotation à proximité du dispositif de couplage distal (600).

9. Dispositif de rotation (6) selon l'une quelconque des revendications précédentes, dans lequel le système optique (63) est disposé dans un évidement (614) d'une section de tige (613) du ensemble fixe (61) et est placé de façon à pouvoir faire tourner le ensemble pivotant (60) autour de la section de tige (613).

10. Dispositif de rotation (6) selon l'une quelconque des revendications précédentes, dans lequel le dispositif de couplage proximal (610) prend la forme d'un entonnoir oculaire.

11. Dispositif de rotation (6) selon la revendication 10, dans lequel le dispositif de couplage proximal (610) prenant la forme d'un entonnoir oculaire est réalisé comme un entonnoir oculaire (12) d'un endoscope (1) à relier au dispositif de couplage distal (600).

12. Dispositif de rotation (6) selon l'une quelconque des revendications précédentes, dans lequel le dispositif d'entraînement (62) comporte un pignon (621) en prise avec une roue dentée (601) du ensemble pivotant (60).

13. Dispositif d'endoscope avec :
un dispositif de rotation (6) selon l'une quelconque des revendications précédentes ;
un endoscope (1) pouvant être relié au dispositif de couplage distal (600) du dispositif de rotation (6) ;
un dispositif de roue de caméra (2) pouvant être relié au dispositif de couplage proximal (610) du dispositif de rotation (6).

14. Dispositif d'endoscope selon la revendication 13, dans lequel l'endoscope (1) comporte une tige (10) et un prisme de basculement (11) disposé au une extrémité (100) opposée au dispositif de rotation (6), pouvant basculer autour d'un axe de basculement (S).
